# EUROPEAN PATENT APPLICATION

(11) **EP 2 221 086 A1**
(43) Date of publication of application: **25.08.2010**
(21) Application number: 09002599.0
(22) Date of filing: 24.02.2009
(51) Int. Cl.: A61N 1/32

(54) **Portable equipment comprising generator circuit and provider for therapeutic transfer of molecules by electroporation**

(71) Applicant: Spugnini, Enrico, 000147 Roma (RM) (IT)
(72) Inventor: Spugnini, Enrico, 00147 Rome (IT); Baldi, Alfonso, 80128 Naples (IT); Aiello, Lorenzo, 00124 Rome (IT); Mudrov, Nikolay, 1408 Sofia (BG); Daskalova, Marina, 1120 Vienne (AT); Dotsinsky, Yvan, 1797 Sofia (BG)
(74) Representative: Hervouet, Sylvie

(57) **Abstract**

The present invention aims to address at least partially the above issues in a much more versatile configuration that will allow more efficient and safer procedures in a broader spectrum of clinical conditions compared to the other electroporators.

To reach this purpose, a portable equipment for therapeutic transfer of molecules according to the invention comprises electroporation protocol means to control biphasic pulses sequences with a parameters set comprising the number of pulses in each sequence, bipolar shape and duration of each pulse component as well as pause duration between two sequences, means to download said parameters preliminary determined as depending of the transfer protocol. The pulses are provided by a circuit comprising a rechargeable battery that powers a capacitor to generate a burst of biphasic pulses to electrodes at a high voltage, requiring only a control on the voltage of the generated pulses.

## Description

### BACKGROUND OF THE INVENTION

The invention relates to portable equipments for therapeutic transfer of molecules, in particular: antiblastics, anti-inflammatories, hormones, cytokines, immune-regulators, antibodies, DNA, etc.

The invention concerns in general electrochemotherapy and gene therapy in animals, in particular for local drug delivery by electroporation.

It is known from the patent document US 6 241 701 an apparatus for electroporation mediated delivery of drugs and genes. This apparatus includes a needle array electrode having a "keying" element that determines the set point of the therapy voltage pulse. However, the apparatus does not built up safeties for the operator, is not efficient and is very cumbersome.

Moreover, electroporation through the application of trains of biphasic pulses is described in several scientific research articles, such as EP, A Porrello "Potentiation of chemotherapy in companion animals with spontaneous large neoplasms by application of biphasic electric pulses", Spugnini et al, J. Exp. Clin. Canc. Res. 2003.

### GENERAL PROBLEM AND PARTICULAR PROBLEM TO BE SOLVED BY THE INVENTION

Human and veterinary oncologists acutely perceive the difficulty of achieving local tumor control in cancer patients affected by various neoplasms. Cancer is considered a disease that requires multi-modality treatment. Chemotherapy is usually combined with surgery and/or radiation therapy treatments in an adjuvant fashion for those cancers with high tendency to metastasize, i.e. high grade sarcoma or breast cancer.

Synergistic combinations led to the investigation of local and whole body hyperthermia. However, the high costs of this treatment as well as the lengthiness of the procedure and the need for highly-skilled operators confined this therapy to a small number of research institutions.

Furthermore, the most commonly reported side effects of radiation therapy are:
1) gradual side-effects, usually dose-dependent (local fibrosis, necrosis, nerve damage etc.) and
2) the so called "statistically demonstrable side effects", also known as "radiation induced tumors".

A new treatment modality being further explored that can achieve high rates of remission without the associated problems of high financial and biological cost of previous procedures is electrochemotherapy (ECT). It involves the administration of anticancer agents to the application of permeabilizing pulses so to increase the uptake of antitumor molecules.
*In vitro* studies showed that the application of high voltage, exponentially-decaying electric pulses to cells in suspension could induce pores in the cell membrane (Microsc Res Tech. 2007 Dec; 70(12):1041-50. Ultrastructural modifications of cell membranes induced by "electroporation" on melanoma xenografts by Spugnini EP *et al*), thus resulting in cross-membrane flow of material (electroporation, electro-injection). These methods were initially used to transfect bacterial cells with plasmids and subsequently exploited to produce monoclonal antibodies through fusion of eukaryotic cells.

Later, researchers realized that electroporation could enhance the transport of drugs and genes through the cytoplasmic membrane by exposing animal cells in culture and plant protoplasts to non-cytotoxic electric pulses. Subsequently, electroporation has been proven to be very effective at enhancing the *in vitro* cytotoxicity of anticancer molecules.

The first and most actively studied chemotherapy agent in ECT has been bleomycin.
This drug can penetrate the cell membrane only through protein receptors due to its lipophobic nature, thus resulting in slow and quantitatively limited uptake under normal conditions. The complex formed by bleomycin and its carrier is transported in the cytosol by means of endocytotic vesicles, but the mechanism of its release is still unknown.

Several electroporation protocols have been adopted, mostly involving sequences of repeated decaying or square single pulses until the desired number of permeabilizing electric stimulations was reached.

Most electrochemotherapy equipments are rather cumbersome and often require a cart to be moved. Moreover, since they are projected for both basic research and clinical activity, have a broad parameter of selections regarding duration of the pulses, number of pulses, and voltage of the pulses. The equipments adapted unidirectional pulses that need the operator to re-orient the electrodes for optimal electroporation (for instance: Sersa G. et al. Clinical Cancer Research 6: 863-867, 2000). They need an external electrical supply and are not suited for field use.

Most equipments deliver single pulses: this leads to significant increase of the duration of the therapeutic sessions and increases morbidity for the patient. Many types of equipment adopt non reusable electrodes. All the electrodes are not suited for intra-operative use.

The known equipments are most painful for the patient that is generally evidenced in electrostimulation for monopolar stimuli compared to bipolar ones.

### SUMMARY OF THE INVENTION

The present invention aims to address at least partially the above issues in a much more versatile configuration that will allow more efficient and safer procedures in a broader spectrum of clinical conditions compared to the other electroporators.

The present equipment is built around discharging a train of biphasic pulses delivered as a burst to electrodes at a high voltage to generate a successful shock.

The parameters choice of the sequence of pulses gives the possibility of voltage control of the pulses to contribute to a successful shock generation of the train of biphasic pulses. The charge is controlled to warn the operator of the status of charge.

More precisely, the object of the present invention is a portable equipment for therapeutic transfer of molecules comprising electroporation protocol means to control biphasic pulses sequences with a parameters set comprising the number of pulses in each sequence, bipolar shape and duration of each pulse component as well as pause duration between two sequences, means to download said parameters preliminary determined as depending of the transfer protocol, the pulses being provided by a circuit comprising a rechargeable battery that powers a capacitor to generate a burst of biphasic pulses to electrodes at a high voltage, requiring only a control on the voltage of the generated pulses.

According to preferred embodiments:
- the equipment is set to deliver an optimal train of limited biphasic pulses, in particular 8 pulses;
- each start of electroporation generates two bipolar pulses (one positive immediately followed by one negative) each of them with a duration, e.g. of 50 µs. Then a pause of, e.g.1 ms, is introduced. This interval is repeated a limited times, for instance 8, until the end of the sequence;
- a particular selection of pulse sequence is provided with the adopted bursts of +50 , -50 µs rectangular biphasic pulses that are repeated with a pause of 1 ms between two consecutive pulses. The rectangular shape is ensured by an impulse transformer;
- in order to deliver any pulse sequency, the capacitor is charged by the battery and then released by the operator: therefore the pulse sequency cannot be accidentally generated thus harming the operator. Thus, a full electrical safety for the patient and the physician is warranted by battery pulse generation and high fidelity battery charging;
- the insulation between the adapter and the applied part (electrode) is achieved by an impulse transformer that meets the requirement for some thousands of volts, for instance 4000 V dielectric strength;
- the transformer has one primary coil only winded up on ferrite core that leads to small dimensions and weight compared to these of a transformer made of dynamo sheet. The full-wave rectification is accomplished by a bridge circuit with low breakdown voltage switches, typically 600V. This design is efficient compared to the traditional coil used with double turns and middle point and the two switches of higher breakdown voltage controlling the rectified voltage;
- additional means against involuntary provoked high voltage shock are taken to protect the patient:
   ○ a microcontroller reduces the eventual initial default pulse voltage up to 100V. Once selected, the electrical charge is available to be applied on the patient not more than a predefined duration, e.g. 10 s. After that, an automatic discharge is provided by the microcontroller for if the shock is not started;
   ○ the microcontroller blocks any accidentally delivering of the pulse sequences when the equipment is reloading the battery;
   ○ the microcontroller disconnects the equipment from the electrical network when operational during pulse sequence generation;
- a warning device is provided to inform of the depletion of the battery;
- the charge of the battery is monitored by a microprocessor and can be displayed to warn the operator when the charge will be almost depleted; after warning, the battery is still able to deliver a limited number of pulses, e.g. at least 10 pulses;
- the delivery of the sequence generation of pulses may be cancelled in any time of the parameter selection by depression control means, in particular by a simple button, a remote controller or through a pedal;
- a display on a monitoring means when switching on the equipment shows the allowed lowest initial default value of 100 V that may be used for pulse sequence generation except for voltage change selected by the operator; capacitor to be automatically discharged when the state is abnormal to block the pulse generation or during the battery charging to enhance the electrical safety and the burst delivery cannot then be started with an initial default voltage;
- the electroporation provider is constituted of PTFE (polytetrafluoroethylene) or PP (polypropylene);
- the provider combines in one set a calliper and electrodes. This combination is specifically projected for the treatment of cancer in companion animals. The electrodes of such providers are then re-usable and can be sterilized allowing intra-operative electrochemotherapy.

The present invention presents the complementary following advantages:
- an easy but optimal selection of appropriate pulse sequence, which is more tolerable by the patient compared to the other equipments for electroporation;
- a short and tolerable but high efficient pulse sequence is used. Its generation is very simple and easy controlled. The sequence parameters are optimally fixed. The physician has only to select the voltage. All other modes of operation, e.g. starting the pulse generation, interruption the generation, safety battery charging, are controlled by means of friendly interacting software;
- biphasic pulses allow a smoother electric transition;
- the pulses are delivered as a burst, thus decreasing the duration of the treatment and the morbidity for the patients;
- the equipment adopts a simplified electroporation protocol: clinician will need only to set the voltage;
- the equipment when operational is aimed to be disconnected from the electrical network.
- the impulse cannot be accidentally delivered when the equipment is recharging its battery;
- the equipment works on a battery and has built up safeties for the operator: the impulse cannot be accidentally generated thus harming the operator. In order to be delivered, the capacitor must be charged by the battery and then released by the operator;
- the capacitor is auto-discharged if the impulse is not delivered within a limited duration, e. g. 10 seconds;
- the equipment is lighter and smaller compared to known electroporators;
- the independency from the electrical supply makes this equipment a reliable portable field instrument for electroporation;
- the time necessary for a full charge of a depleted battery is limited, e.g. 12 hours;
- the instrument fully charged will have autonomy of more than 200 electroporations, e.g. 240 electroporations;
- the transformer sizes allowed the building of the portable equipment with small dimensions and weight but in the same time capable to generate more than 200 shocks with the maximum voltage of substantially 2200 V. Due to the relatively lower but efficient energy delivered to the patient, the battery is capable to generate more than 200 shocks with the maximum voltage of 2200 V before being recharged;
- the burst delivery ***is*** started with the lowest initial default value of 100 V shown on the display when switching on the equipment, since higher voltage may be not useful for electroporation and dangerous for the patient. The pulse generation can be blocked also during battery charging to enhance the electrical safety of both the patient and physician;
- two factors contribute to the portability of the electroporator: the efficient pulse sequence with relatively low energy charged in small capacitor and the reduced transformer sizes;
- the train of limited biphasic pulses delivers low energy, which is enough for a successful electroporation. In the same time, it is more tolerable to the patient because of the bipolar shape used. Furthermore, the sequence of very near pulses is practically felt as one high voltage shock and not as many single shocks;
- the equipment has a simplified electroporation protocol means: the electroporation is one shot controlled and the operator has only to select the pulse voltage.

Furthermore, the proposed electroporation provider is characterised both in material, PTFE or PP, and in combining in one set calliper and needle arrays. This combination is specifically projected for the treatment of cancer in companion animals. The electrodes of such providers are then re-usable and can be sterilized allowing intra-operative electrochemotherapy.

### BRIEF DESCRIPTION OF THE FIGURES

- Fig. 1, a schematic block diagram of a pulse generator for electroporation according to the invention;
- Fig. 2, an example of a bridge circuit of such a generator;
- Fig. 3a to 3d, four different views (respectively: perspective, lateral, front and upper views) of an electroporation provider with needles as electrodes;
- Fig. 4a and 4b, alternative gratings shapes for the electrodes, and
- Fig.5a and 5b, perspective views of handlers for a provider.

### DETAILED DESCRIPTION OF AT LEAST ONE EMBODIMENT

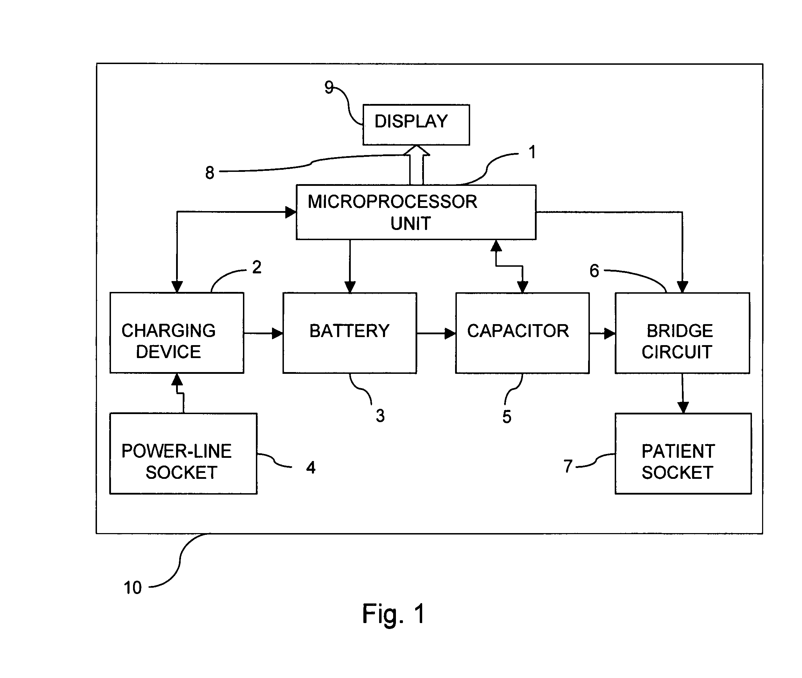

Fig. 1 shows a schematic block diagram of an example of pulse generator 10 according to the invention. In this generator, a microprocessor unit 1 controls a charging device 2, which connects and disconnects a battery 3 from the power-line socket 4. The microprocessor unit 1 controls also the charge of a capacitor 5 and a bridge circuit 6, which has an output to the patient socket 7. A bus 8 of the microprocessor unit 1 is fed to a display monitor 9, which facilitates the parameters choice to e implemented.

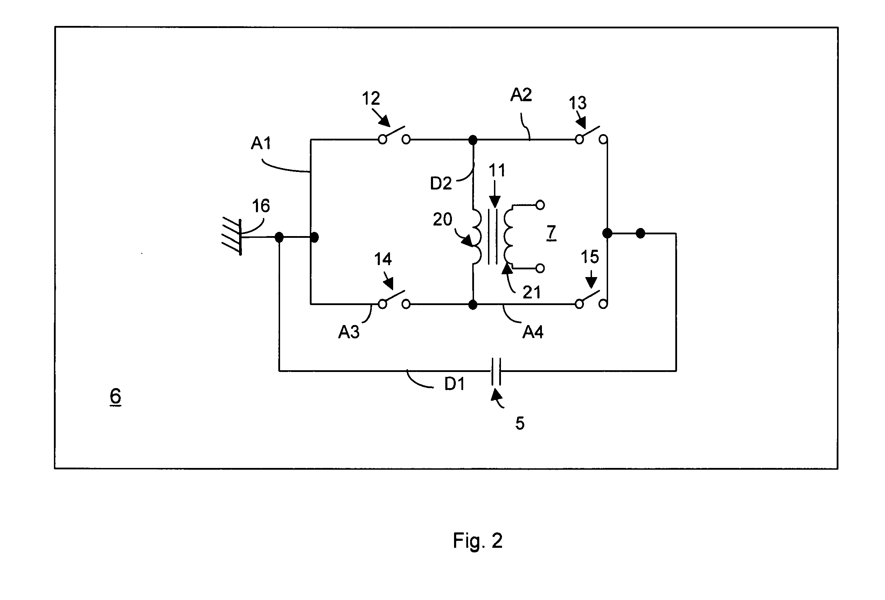

Fig. 2 depicts in more details the structure of the bridge circuit 6 of Fig.1. The charged capacitor 5 is included in a first diagonal D1 of the bridge circuit 6 and the second diagonal D2 consists of the primary coil 20 of a transformer 11. Each arm A1-A4 of the bridge includes respectively one of the high voltage switches 12-15. The switches are connected to achieve pairs of turned on/off: switch 12 with switch 15 and switch 13 with switch 14 to change the polarity of the output pulses at secondary coil 21 of the transformer 11 where the patient socket 7 is connected.

The primary coil 20 of the transformer 11 is smaller than a coil with a middle point controlled by two switches. The used switches 12-15 are subjected to lower voltages and can be selected among the low cost components for every use. The small transformer 11 size reduces the loss due to the scattering field and limits the leakage current through the patient, who is highly insulated from the earth 16. Thus the patient safety is increased.

In the present example, the equipment provides for a selection of appropriate pulse sequence of 50 plus 50 µs rectangular biphasic pulses that are repeated with a pause of 1 ms between two consecutive pulses. The rectangular shape is ensured by the impulse transformer 11.

The sequence generation is trigged by a control system but its start may be cancelled by the operator in any time of the parameter selection.

The sequence generation is triggered by action on a front panel. Actionners on front and panels for switching the instrument, for voltage choice and for shock generation. They transmit signals to the microprocessor unit, which controls the electroporation.

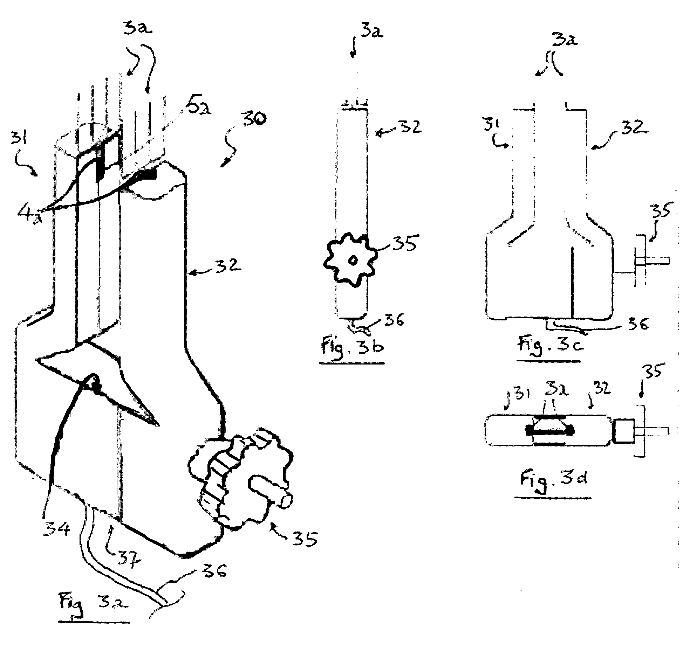

Each provider 30 is built up with two long-nose handlers 31 and 32, as illustrated by Fig.3a to Fig.3d. The handlers are joined in a scaffold spring pliers-like forming a calliper, by means of a screw 34 and locked with a rotating knob 35. Needle arrays 3a acting as electrodes are inserted by its basis 4a in a recess 5a of each handler. Electrical wires 36 of the electrodes are connected to the pulse generator at the bottom 37 of the handlers.

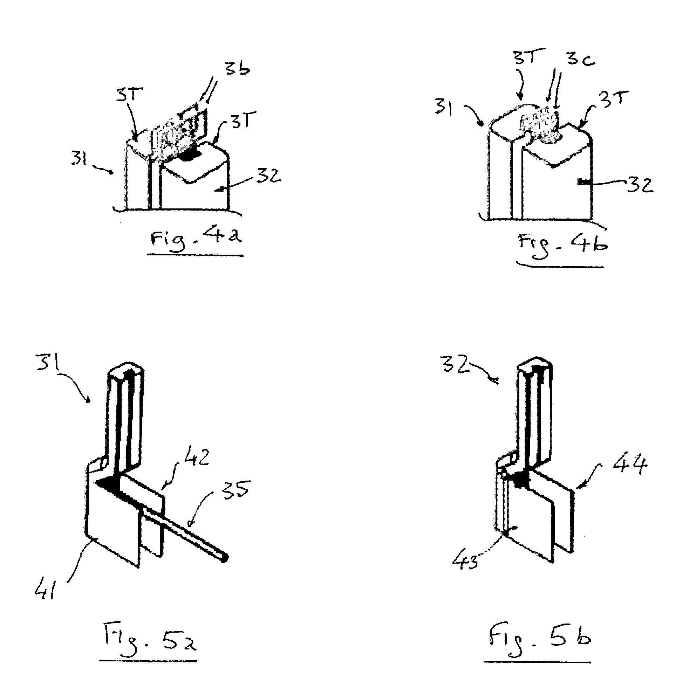

Alternatively, as shown in Fig. 4a and 4b, gratings 3b and 3c can also act as electrodes inserted at the top 3T of the handlers 31, 32 to complete the apparatus. The handlers can be easily disassembled in order to allow the changing of selected electrodes.

As better shown on perspective views of Fig. 5a and 5b, one handler 31 has endless screw 35 and two lateral jaws 41, 42. The other handler 32 has also two lateral jaws 43, 44 that fit into each other regarding the respective jaws 41, 42 when the handlers are joined.

The handlers are therefore built to prevent the rotation of the inserted parts thus allowing a proper alignment of the needles 3a or gratings 3b and maintain a homogeneous electrical field.

Just the metallic electrodes come into contact with the patient skin and biological fluids. They are removable, but linked to the electrical pulse generator by electrical contacts (not represented) running on the inner side of the handlers from top to bottom and - by means of a connection - by electrical wires.

With the exception of the electrodes, the whole equipment cannot extend the electrical discharge while pulsing, being electrical wires adequately covered and handlers constituted in PTFE (Teflon®) to prevent oxidation and corrosion and that is resistant to sterilization procedures in autoclave.

In the example, the endless screw 35 within the handler 31 allows a movement of the two jaws 41, 42 from a minimal distance of 5 mm up to a maximal distance of 30 mm. A contrast spring around the screw will allow a more precise positioning of the handlers and electrodes mounted on the handlers. The electrical circuit prevents overheating or short-circuits.

## Claims

1. Portable equipment for therapeutic transfer of molecules comprising electroporation protocol means to control biphasic pulses sequences with a parameters set comprising the number of pulses in each sequence, bipolar shape and duration of each pulse component as well as pause duration between two sequences, means to download said parameters preliminary determined as depending of the transfer protocol, the pulses being provided by a circuit comprising a rechargeable battery that powers a capacitor to generate a burst of biphasic pulses to electrodes at a high voltage, requiring only a control on the voltage of the generated pulses.

2. Portable equipment according to claim 1, wherein the generator circuit is set to deliver a train of a limited biphasic pulses.

3. Portable equipment according to claim 2, wherein the generator circuit delivers a train of 8 biphasic pulses.

4. Portable equipment according to anyone of the preceding claim, wherein each start of the generator circuit provides two bipolar pulses, one positive immediately followed by one negative, each of them with a fixed duration, followed by a pause, this interval being repeated a limited times until the end of the sequence in order to constitute said train.

5. Portable equipment according to claim 4, wherein a particular selection of pulse sequences is provided with bursts of 50 + 50 µs rectangular biphasic pulses achieved by an impulse transformer of the circuit (6) that are repeated 8 times with a pause of 1 ms between two consecutive pulses.

6. Portable equipment according to anyone of the preceding claim, wherein the battery providing the burst of pulses is charged through a power-line adapter that allows this charge only when all functions of the equipment are blocked.

7. Portable equipment according to anyone of the preceeding claims, wherein the insulation between the adapter and the electrodes is achieved by an impulse transformer that meets the requirement for some thousand volts dielectric strength.

8. Portable equipment according to the preceding claim, wherein the transformer has one coil only winded up on ferrite core that leads to reduce dimensions and weight, the full-wave rectification being accomplished by a bridge circuit with breakdown voltage switches, typically 600 V.

9. Portable equipment according to anyone of the preceding claim, wherein an additional microcontroller against involuntary provoked high voltage shock reduces the eventual initial default pulse voltage up to 100 V and causes, after a predetermined duration, an automatic discharge if the shock is not started.

10. Portable equipment according to anyone of the preceding claim, wherein a warning device provides information of the depletion of the battery.

11. Portable equipment according to anyone of the preceding claim, wherein the charge of the battery is monitored and can be displayed to warn the operator when the charge is almost depleted.

12. Portable equipment according to anyone of the preceding claim, wherein the delivery of the sequence generation of pulses may be cancelled in any time of the parameter selection by depression control means.

13. Portable equipment according to the preceding claim, wherein the depression control means is chosen between a remote controller, a button and a pedal.

14. Portable equipment according to anyone of the preceding claim, wherein a display on a monitoring means shows the allowed lowest initial value of 100 V that is used for the pulse sequence generation except for voltage change caused by the operator.

15. Portable equipment according to anyone of the preceding claim, wherein the electroporation provider is constituted of PTFE or PP.

16. Portable equipment according to anyone of the preceding claim, wherein the provider combines in one set a calliper and electrodes.

## Amended claims

### Amended claims in accordance with Rule 137(2) EPC.

**1.** Portable equipment for therapeutic transfer of molecules comprising:
- an electroporation provider (30) with electrodes (7; 3a, 3b);
- a generator circuit (10) for generating a burst of biphasic pulses to said electrodes at a high voltage according to an electroporation protocol to control biphasic pulses sequences with a downloaded parameters set comprising the number of pulses in each sequence, bipolar shape and duration of each pulse component as well as pause duration between two sequences,
**characterized in that** said generator circuit (10) comprises:
- a bridge circuit (6) with four arms (A1, A2, A3, A4), including a capacitor (5) in a first diagonal (D1) of the bridge circuit (6), and a primary coil (20) of an impulse transformer (11) in a second diagonal (D2) of said bridge circuit (6), and four switches (12-15) connected to achieve pairs of turned on/off, wherein the secondary coil (21) of said impulse transformer delivers output to said electrodes;
- a rechargeable battery (3) to power said capacitor (5) and
- a microprocessor unit (1) for controlling the charge of said capacitor and said bridge circuit (6).

**2.** Portable equipment according to claim 1, wherein the generator circuit (10) is set to deliver a train of a limited biphasic pulses.

**3.** Portable equipment according to claim 2, wherein the generator circuit (10) delivers a train of 8 biphasic pulses.

**4.** Portable equipment according to anyone of claims 2 to 3, wherein each start of the generator circuit (10) provides two bipolar pulses, one positive immediately followed by one negative, each of them with a fixed duration, followed by a pause, this interval being repeated a limited times until the end of the sequence in order to constitute said train.

**5.** Portable equipment according to claim 4, wherein a particular selection of pulse sequences is provided with bursts of 50 + 50 µs rectangular biphasic pulses achieved by an impulse transformer (11) of the circuit (6) that are repeated 8 times with a pause of 1 ms between two consecutive pulses.

**6.** Portable equipment according to anyone of the preceding claims, wherein the battery (3) providing the burst of pulses is charged through a power-line adapter (2) that allows this charge only when all functions of the equipment are blocked.

**7.** Portable equipment according to claim 6, wherein the insulation between the power-line adapter (2) and the electrodes (7; 3a, 3b) is achieved by said impulse transformer (11) that meets the requirement for some thousand volts dielectric strength.

**8.** Portable equipment according to anyone of the preceding claims, wherein an additional microcontroller against involuntary provoked high voltage shock reduces the eventual initial default pulse voltage up to 100 V and causes, after a predetermined duration, an automatic discharge if the shock is not started.

**9.** Portable equipment according to anyone of the preceding claims, wherein a warning device provides information of the depletion of the battery (3).

**10.** Portable equipment according to anyone of the preceding claims, wherein the charge of the battery (3) is monitored and can be displayed to warn the operator when the charge is almost depleted.

**11.** Portable equipment according to anyone of the preceding claims, wherein the delivery of the sequence generation of pulses may be cancelled in any time of the parameter selection by depression control means.

**12.** Portable equipment according to the preceding claim, wherein the depression control means is chosen between a remote controller, a button and a pedal.

**13.** Portable equipment according to anyone of the preceding claims, wherein a display (9) on a monitoring means shows the allowed lowest initial value of 100 V that is used for the pulse sequence generation except for voltage change caused by the operator.

**14.** Portable equipment according to anyone of the preceding claims, wherein the electroporation provider (30) is constituted of PTFE or PP.

**15.** Portable equipment according to anyone of the preceding claim, wherein the electroporation provider (30) combines in one set a calliper and electrodes.
